# EUROPEAN PATENT APPLICATION

(11) **EP 4 553 492 A1**
(43) Date of publication of application: **14.05.2025**
(21) Application number: 24764112.9
(22) Date of filing: 08.02.2024
(51) Int. Cl.: G01N 23/223, G01N 23/2202, G01N 23/2204, H01M 4/04, H01M 4/139

(54) **DEVICE AND METHOD FOR DETECTING FOREIGN MATTER IN ELECTRODE ACTIVE MATERIAL**

(30) Priority: 27.02.2023 KR 20230026212
(71) Applicant: LG Energy Solution, Ltd., Seoul 07335 (KR)
(72) Inventor: SON, Young Ki, Daejeon 34122 (KR); MOON, Hee Chul, Daejeon 34122 (KR)
(74) Representative: Goddar, Heinz J.
(86) International application number: PCT/KR2024/001874
(87) International publication number: WO 2024/181712

(57) **Abstract**

The present technology provides a device for detecting foreign substances in an electrode active material including: a suspension tank containing a suspension in which an electrode active material is uniformly dispersed; a filter having a predetermined pore size, and configured to filter the suspension transferred from the suspension tank to collect foreign substances in the electrode active material; and an analyzer configured to analyze the filter in which the foreign substances are collected to detect at least one of types, a total number, shapes, and sizes of the foreign substances. The present technology also provides a method of detecting foreign substances in an electrode active material.

## Description

### [Technical Field]

The present invention relates to a device and method for detecting foreign substances in an electrode active material.

More particularly, the present invention relates to a device and method for detecting foreign substances in an electrode active material at a high detection rate.

This application claims the benefit of priority based on Korean Patent Application No. 10-2023-0026212, filed on February 27, 2023, and the entire content of the Korean patent application is incorporated herein by reference.

### [Background Art]

A secondary battery is manufactured by putting an electrode assembly in a battery case and injecting an electrolyte. The electrode assembly has a structure in which a positive electrode and a negative electrode are stacked with a separator interposed therebetween.

An electrode such as a positive or negative electrode is manufactured by coating a current collector with an active material slurry. The electrode active material may contain a small amount of foreign substances. Foreign substances in an electrode may cause a low voltage defect and deteriorate the characteristics and quality of a secondary battery cell manufactured using the electrode.

Therefore, it is important to identify foreign substances that affect the characteristics of the secondary battery by detecting the types, total number, shapes, sizes, etc. of foreign substances in the electrode active material. To this end, the foreign substances in the electrode active material should be collected and detected at a high probability. **In** addition, it is preferable to inspect a large amount of the electrode active material as quickly as possible to match a production speed of mass production facility.

One of conventional methods of detecting foreign substances in an active material is a method of filtering electrode active material powder by applying air pressure to upper and lower sides of a metal filter. Active material powder that is smaller in size than foreign substances passes through the metal filter, and foreign substances are collected on a surface of the metal filter. Because foreign substances are dispersed in an entire area of a filter due to air pressure, a filter with a large area is required to collect the foreign substances. Because foreign substances are sparsely collected in a filter with a large area, collection density is increased by repeatedly attaching and detaching a tape to or from the filter several times. The tape to which foreign substances are attached may be analyzed by, for example, an X-ray fluorescence (XRF) analyzer to identify the types of the foreign substances.

However, in this method, a large amount of foreign substances may be lost during the adhering and detaching of the tape and thus the amount or number of foreign substances in the active material cannot be accurately identified. **In** addition, because the metal filter is used, foreign substances may not be appropriately adsorbed on the metal filter due to magnetic properties, e.g., weak magnetic properties or paramagnetic properties, of the foreign substances. Thus, a detection rate (recovery rate) of foreign substances in the electrode active material is very low, and thus this method is very difficult to apply to detection of foreign substances.

To improve the problem, there is a method of sampling electrode active material powder containing foreign substances onto a film and analyzing the sampled electrode active material powder by an analyzer. However, in this case, the foreign substances in an electrode active material were detected together with an active material and thus it was difficult to inspect a large amount of active material. Only a small amount, e.g., about 10 mg, of samples can inspected. To inspect a large amount of active material, a sampling area should be increased greatly. However, the XRF analyzer is limited in terms of a set resolution and scan speed. Therefore, it takes a long time to scan a sample film with a large area at a set resolution and scan speed. Therefore, such a detection method is applicable only to experiments conducted at a laboratory level, and it is difficult to quickly inspect a large amount of samples to meet mass production environments of factories.

### [Related Art Literature]

### [Patent Document]

Korean Patent Publication No. 10-2014-0048370

### [Disclosure]

### [Technical Problem]

To address the above-described problems, the present invention is directed to providing a foreign substance detecting device and method for detecting foreign substances in an electrode active material at a high detection rate.

The present invention is also directed to providing a foreign substance detecting device and method for quickly inspecting a large amount of electrode active material.

### [Technical Solution]

An aspect of the present invention provides a device for detecting foreign substances in an electrode active material, the device including: a suspension tank containing a suspension in which an electrode active material is uniformly dispersed; a filter having a predetermined pore size, and configured to filter the suspension transferred from the suspension tank to collect foreign substances in the electrode active material; and an analyzer configured to analyze the filter in which the foreign substances are collected to detect at least one of types, a total number, shapes, and sizes of the foreign substances.

**In** an embodiment, the suspension tank may include an agitating member configured to agitate and disperse the electrode active material and the foreign substances.

**In** an embodiment, the suspension may be transferred to the filter at a constant flow rate.

For example, the suspension may be transferred to the filter and filtered by the filter while being sealed from the outside.

For example, the device may further include a foreign substance collection kit sealed from the outside except for a liquid inlet and a liquid outlet, and the filter may be installed in a communication channel between the liquid inlet and the liquid outlet.

The liquid inlet of the foreign substance collection kit and the suspension tank may be connected through an airtight conduit.

In an embodiment, the device may further include a peristaltic pump installed in the sealed conduit between the suspension tank and the foreign substance collection kit to transfer the suspension at a constant flow rate.

The filter may be a polymer filter.

For example, the analyzer may be an X-ray fluorescence (XRF) analyzer configured to analyze the foreign substances qualitatively and quantitatively from secondary X-rays generated for types of the foreign substances by emitting X-rays to the filter in which the foreign substances are collected.

An area of a foreign substance collection region of the filter may be set to complete X-ray scanning by the XRF analyzer within a predetermined time under set resolution and scan speed conditions of the XRF analyzer.

Another aspect of the present invention provides a method of detecting foreign substances in an electrode active material, the method including: preparing a suspension by suspending and agitating an electrode active material in a liquid, wherein the electrode active material is uniformly dispersed in the suspension; collecting foreign substances, which are contained in the electrode active material, in the filter by passing the suspension through a filter with a predetermined pore size; and analyzing the filter in which the foreign substances are collected to detect at least one of types, a total number, shapes, and sizes of the foreign substances.

The suspension may be transferred to the filter at a constant flow rate.

For example, the suspension may be transferred to the filter and filtered by the filter while being sealed from the outside.

In the method, at least one of types and total number of the foreign substances may be detected by an XRF analyzer that analyzes the foreign substances qualitatively and quantitatively from secondary X-rays generated for the types of the foreign substances by emitting X-rays to the filter in which the foreign substances are collected.

In the method, an area of a foreign substance collection region of the filter may be set to complete X-ray scanning by the XRF analyzer within a predetermined time under set resolution and scan speed conditions of the XRF analyzer.

### [Advantageous Effects]

According to the present invention, foreign substances in an electrode active material can be detected with a high probability. That is, the foreign substances in the electrode active material can be detected at a high detection rate (recovery rate).

According to the present invention, foreign substances can be detected by analyzing a large amount of electrode active material.

In addition, according to the present invention, an electrode active material can be analyzed within a short time by taking into account the resolution or scan speed of an analyzer, thereby rapidly detecting foreign substances.

### [Brief Description of the Drawings]

FIG. 1 is a schematic view of a device for detecting foreign substances in an electrode active material according to a first embodiment of the present invention.
FIGS. 2 and 3 are a schematic view and a cross-sectional view of a foreign substance collection kit included in the foreign substance detecting device.
FIGS. 4 and 5 are a schematic view and a cross-sectional view of another example of a foreign substance collection kit included in the foreign substance detecting device.
FIG. 6 is a schematic view of an area of a foreign substance collection region of a filter applied to the present invention.
FIG. 7 is a schematic view of a state in which a filter is attached to a film to be inspected.
FIG. 8 is a schematic view of a device for detecting foreign substances in an electrode active material according to a second embodiment of the present invention.
FIG. 9 is a schematic diagram illustrating an operation of a peristaltic pump.
FIG. 10 is a flowchart of a method of detecting foreign substances in an electrode active material according to the present invention.
FIG. 11 is a mapping map for foreign substances, obtained by an X-ray fluorescence (XRF) analyzer.
FIG. 12 is an image of foreign substances that is obtained by a microscope attached to an XRF analyzer.
FIG. 13 is a graph showing a correlation between the amount of foreign substance powder and the number of foreign substances.

### [Best Mode]

Hereinafter, the present invention will be described in detail. Before describing the present invention, the terms or expressions used in the present specification and claims should not be construed as being limited to as generally understood or as defined in commonly used dictionaries, and should be understood according to meanings and concepts matching corresponding to the present invention on the basis of the principle that the inventor(s) of the application can appropriately define the terms or expressions to optimally explain the present invention.

It should be understood that the terms "comprise" and/or "comprising", when used herein, specify the presence of stated features, integers, steps, operations, elements, components, or a combination thereof, but do not preclude the presence or addition of one or more features, integers, steps, operations, elements, components, or a combination thereof. It should be understood that when an element or part is referred to as being "connected" to another element or part, the element or part is connected directly or indirectly to the other element or part.

As used herein, terms such as "on" or "below", "front" or "back", or "dispose" or "arrange" are not intended to limit the invention and should be interpreted as providing examples of terms indicating positions or orientations.

Hereinafter, the present invention will be described in detail.

### <Foreign Substance Detecting Device>

### (First Embodiment)

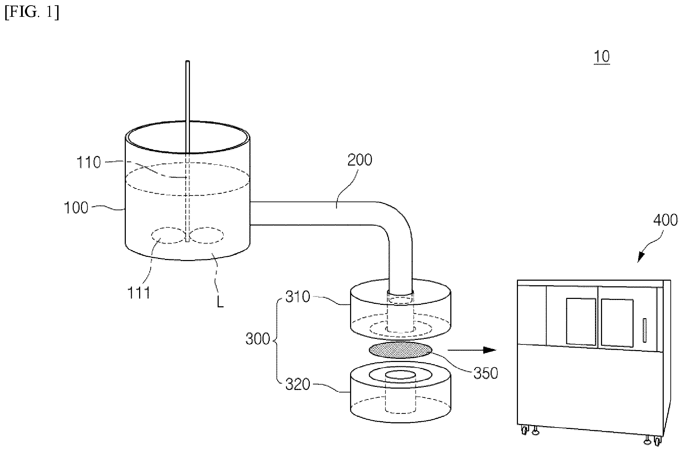

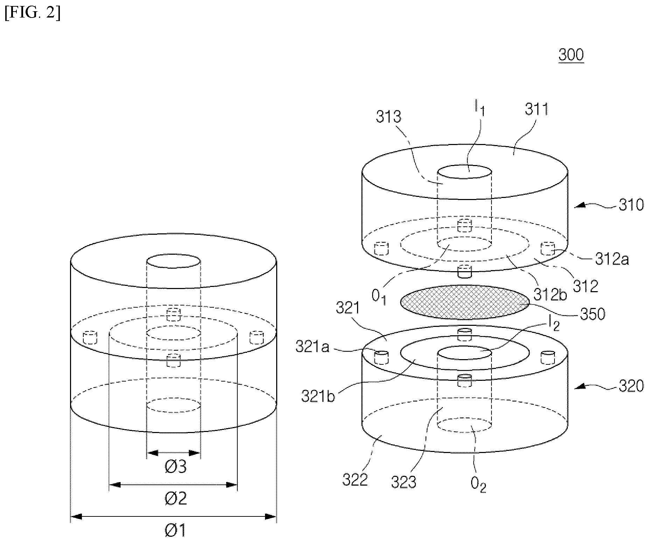

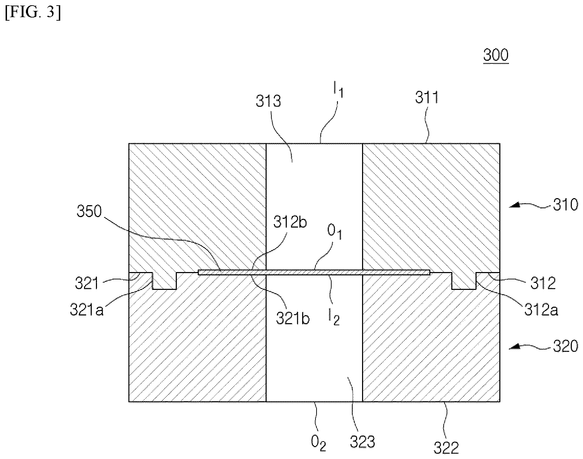

FIG. 1 is a schematic view of a device for detecting foreign substances in an electrode active material according to a first embodiment of the present invention. FIGS. 2 and 3 are a schematic view and a cross-sectional view of a foreign substance collection kit included in the foreign substance detecting device.

The foreign substance detecting device 10 of the first embodiment of the present invention includes: a suspension tank 100 containing a suspension L in which an electrode active material is uniformly dispersed; a filter 350 having a predetermined pore size and configured to filter the suspension L transferred from the suspension tank 100 to collect foreign substances in the electrode active material; and an analyzer 400 configured to analyze the filter 350 in which the foreign substances are collected to detect at least one of the type, total number, shape, and size of the foreign substances.

The electrode active material may include a positive electrode active material and a negative electrode active material. The positive electrode active material may be a lithium-containing oxide, and the lithium-containing oxide may be a lithium-containing transition metal oxide.

For example, the lithium-containing transition metal oxide may include one selected from the group consisting of LiₓCoO₂(0.5<x<1.3), LiₓNiO₂(0.5<x<1.3), LiₓMnO₂(0.5<x<1.3), LixMn₂O₄(0.5<x<1.3), Liₓ(NiₐCo_{d}Mn_{c})O₂(0.5<x<1.3, 0<a<1, 0<b<1, 0<c<1, a+b+c=1), LiₓNi_{1-y}Co_{y}O₂(0.5<x<1.3, 0<y<1), LiₓCo_{1-y}Mn_{y}O₂(0.5<x<1.3, 0≤y<1), LiₓNi_{1-y}Mn_{y}O₂(0.5<x<1.3, 0≤y<1), Liₓ(NiₐCo_{d}Mn_{c})O₄(0.5<x<1.3, 0<a<2, 0<b<2, 0<c<2, a+b+c=2), LiₓMn_{2-z}Ni_{z}O₄(0.5<x<1.3, 0<z<2), LiₓMn_{2-z}Co_{z}O₄(0.5<x<1.3, 0<z<2), LiₓCoPO₄(0.5<x<1.3) and LiₓFePO₄(0.5<x<1.3), or a mixture of two or more of them. The lithium-containing transition metal oxide may be coated with a metal such as aluminum (Al) or a metal oxide. At least one of sulfide, selenide, and halide may be used in addition to the lithium-containing transition metal oxide.

The negative electrode active material may include a carbon material, a lithium metal, silicon, tin, or the like. When a carbon material is used as the negative electrode active material, both low-crystalline carbon and high-crystalline carbon may be used. Representative examples of low-crystalline carbon include soft carbon and hard carbon, and representative examples of high-crystalline carbon include kish graphite, pyrolytic carbon, mesophase-pitch-based carbon fiber, mesocarbon microbeads, mesophase pitches, and high-temperature calcined carbon such as petroleum- or coal-tar-pitch-derived cokes.

It is inevitable that foreign substances are mixed into the electrode active material during the preparation of an active material. There are various types of metal foreign substances as representative examples of foreign substances. For example, there are metal foreign substances such as Fe, Cu, Cr, Zn, Mn, Co, Ni, and Ti. Mn, Co, Ni, and the like may constitute an active material in the form of a compound. Free metal elements that remain after the formation of the compound may be foreign substances that are not involved in an electrical reaction. Particularly, there are a lot of such metal foreign substances in the positive electrode active material. When a secondary battery is manufactured using electrodes containing foreign substances, unexpected defects such as a low-voltage defect may occur. Foreign substances are smaller in size than materials of the electrode active material. Accordingly, the foreign substances in the active material may be collected by the filter 350 of a pore size less than or equal to the sizes of the foreign substances. The electrode active material smaller than the foreign substances may be separated from the foreign substances while passing through the filter 350. The metal foreign substances may be adsorbed and collected by a magnetic member.

As described above, in the related art, foreign substances are detected by a dry method of preparing an electrode active material in the form of powder and filtering the electrode active material to detect metal foreign substance powder. However, in the related method, a foreign substance loss rate is high and thus a foreign substance detection rate is very low. In addition, it is difficult to detect foreign substances by quickly inspecting a large amount of electrode active material to meet a mass production rate of secondary batteries.

In the present invention, foreign substances are detected by a so-called wet detection method of preparing the suspension L by suspending an electrode active material in a liquid, and continuously passing the suspension L through the filter 350 to collect foreign substances.

To this end, the foreign substance detecting device 10 of the present invention includes the suspension tank 100, the foreign substance collection filter 350, and the foreign substance analyzer 400.

The suspension L in which the electrode active material is uniformly dispersed is contained in the suspension tank 100. The suspension L is a mixture of suspension in which small grains do not dissolve but are spread in a liquid as in muddy water. That is, in the suspension L of the electrode active material, the active material and the foreign substances contained in the active material are spread in a liquid in an undissolved state. Accordingly, when the suspension L is passed through the foreign substance collection filter 350, the foreign substances may be collected in the filter 350.

FIG. 1 illustrates the suspension tank 100 that includes an agitating member 110 to agitate the electrode active material and the foreign substances to uniformly disperse them.

The suspension tank 100 is a type of container in which the electrode active material is suspended and agitated in a liquid to uniformly disperse the electrode active material. In FIG. 1, the agitating member 110 includes a rotary shaft rotated by a driving source such as a motor that is not shown, and an agitating blade 111 coupled to the rotary shaft. However, the structure and shape of the agitating member 110 are not limited thereto, and other types of agitating members capable of appropriately agitating a liquid may also be employed.

The liquid in which the electrode active material is suspended may be a liquid in which the active material and the foreign substances do not dissolve but may float in suspension in the liquid. For example, reverse osmosis (RO) water or deionized (DI) water may be used as a liquid for suspension. The RO water is pure water prepared by filtering salt from water by applying pressure by a reverse osmosis treatment device. DI water is water from which ions are removed using an ion exchange resin. That is, purified water obtained by removing impurities, such as ions, solid particles, microorganisms, and organic materials, from water may be used as a liquid for suspension.

The suspension L in which the active material and the foreign substances are uniformly dispersed may be obtained by agitation. Therefore, when the suspension L in which samples are uniformly dispersed is passed through the filter 350, a large amount of samples can be filtered without clogging the filter 350. In particular, a large amount of foreign substances may be stacked and collected by the filter 350 with a limited area by continuously transferring the suspension to the filter 350 at a regular flow rate. According to an example embodiment, according to the present invention, it is possible to analyze a large amount, e.g., 50 to 100 g, of electrode active material can be analyzed. However, the amount of the active material is not limited thereto. Compared to the method of the related art in which only about 10 mg of sampling is possible, a large amount of active material that is hundreds to thousands of times or more than in the related art can be analyzed quickly according to the present invention.

The filter 350 filters a suspension transferred from the suspension tank 100, passes small-sized active material particles, and collects only large-sized foreign substances. To this end, the filter 350 may have a pore size corresponding to the sizes of foreign substances to be collected. For example, the filter 350 may have a pore size of 45 µm to collect metal foreign substances of a length or diameter of 45 µm or more. Alternatively, the filter 350 may have a pore size of 65 µm to collect metal foreign substances of a length or diameter of 65 µm or more.

The filter 350 may comprise a material suitable for filtering, e.g., non-woven fabric, polymer, or stainless steel. However, it may be desirable to use a filter of a material other than a metal, because a metal filter may cause magnetic repulsion against metal foreign substances with weak or paramagnetic properties. For example, a polyethylene (PE) filter may be used as a polymer filter.

To prevent contamination of the suspension, the suspension in the suspension tank 100 may be transferred to the filter 350 while being sealed from the outside. In addition, in order to prevent contamination from the outside in a filtering process, the filter 350 need be installed in an environment sealed from the outside.

To this end, the foreign substance detecting device 10 of the present invention may include an airtight conduit 200 and a foreign substance collection kit 300.

Referring to FIG. 1, the airtight conduit 200 is connected between the suspension tank 100 and the foreign substance collection kit 300. A sealing member (e.g., an O-ring) may be installed at a connection part between the airtight conduit 200 and the suspension tank 100 and a connection part between the airtight conduit 200 and the foreign substance collection kit 300. By preventing leakage of the suspension using the airtight conduit 200, an appropriate amount of the suspension may be transferred to the foreign substance collection kit 300. In addition, it is possible to prevent the mixing of unnecessary bubbles that interfere with the collection of foreign substances by preventing the inflow of external air through the airtight conduit 200. A pump may be employed to transfer a suspension at a regular flow rate from the suspension tank 100 to the filter 350.

The airtight conduit 200 may be formed of a flexible material. For example, the airtight conduit 200 may be a tube or hose formed of flexible plastic resin (e.g., urethane resin) or silicone resin. By bending a flexible hose, a transfer path of the suspension may be freely set between the suspension tank 100 and the filter 350.

The foreign substance collection kit 300 is a type of filter fixture configured to protect the filter 350 from external influences and fix the filter 350 at a predetermined position to collect foreign substances in a specific area of the filter 350.

Referring to FIGS. 2 and 3, the foreign substance collection kit 300 includes a liquid inlet I1, a liquid outlet O2, and communication channels 313 and 323 connecting the liquid inlet I1 and the liquid outlet O2. The foreign substance collection kit 300 is configured to be sealed from the outside except for the liquid inlet I1 and the liquid outlet O2. Therefore, the filter 350 may collect foreign substances stably while being protected from external environments in the foreign substance collection kit 300. The filter 350 is installed in the communication channels 313 and 323.

The liquid inlet I1 of the foreign substance collection kit 300 and the suspension tank 100 are connected by an airtight conduit 200. As described above, a sealing member such as an O-ring may be installed at a connection part between the liquid inlet I1 and the airtight conduit 200.

Referring to FIGS. 2 and 3, the foreign substance collection kit 300 includes: an upper kit part 310 that includes a hollow body with a first hollow channel 313, both ends of which are respectively open toward an upper surface 311 and a lower surface 312 of the hollow body to form a first inlet I1 and a first outlet O1; and a lower kit part 320 that includes a hollow body with a second hollow channel 323, both ends of which are respectively open toward an upper surface 321 and a lower surface 322 of the hollow body to form a second inlet I2 and a second outlet O2. The first inlet I1 and the second outlet O2 correspond to the liquid inlet I1 and the liquid outlet O2 of the foreign substance collection kit 300, respectively. In addition, the first and second hollow channels 313 and 323 are communication channels of the foreign substance collection kit 300.

The foreign substance collection filter 350 may be fixed between the upper kit part 310 and the lower kit part 320 by combining the upper and lower kit parts 310 and 320 such that the first hollow channel 313 and the second hollow channel 323 are concentrically disposed.

The first inlet I1, the first hollow channel 313 and the first outlet O1, and the second inlet I2, the second hollow channel 323 and the second outlet O2 are provided concentrically. The airtight conduit 200 is connected to the first inlet I1 which is a liquid injection port. Therefore, the suspension flowing into the airtight conduit 200 is transferred to the filter 350 through the first inlet I1, the first hollow channel 313, and the first outlet O1. A liquid (mainly containing an electrode active material) filtered by the filter 350 is discharged downward through the second inlet I2, the second hollow channel 323 and the second outlet O2. As described above, the foreign substance collection kit 300 is configured to pass the suspension from top to bottom, so that the suspension can be smoothly filtered due to gravity. Diameters and lengths of the first and second hollow channels 313 and 323 may be appropriately determined in consideration of a flow rate and velocity of the suspension. For example, a length of an entire communication channel, including the first and second hollow channels 313 and 323, may be determined to be in a range of 45 to 70 mm but is not limited thereto. Accordingly, a total height of the foreign substance collection kit 300, including the upper kit part 310 and the lower kit part 320, may be determined to be in a range of 45 to 70 mm but is not limited thereto.

Diameters of the first inlet I1, the first hollow channel 313 and the first outlet O1, and diameters of the second inlet I2, the second hollow channel 323 and the second outlet O2 may be the same. However, as described below, the diameters of the first and second hollow channels 313 and 323 may vary in a height direction.

FIG. 2 illustrates a diameter φ1 of a foreign substance collection kit, diameters φ2 of a filter installed in the foreign substance collection kit and a filter mounting groove, and diameters φ3 of first and second hollow channels and a foreign substance collection region.

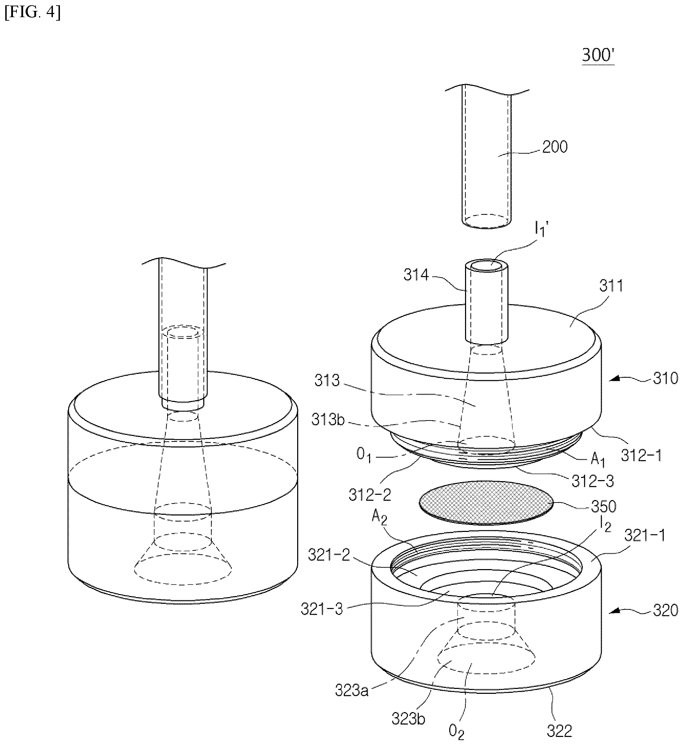

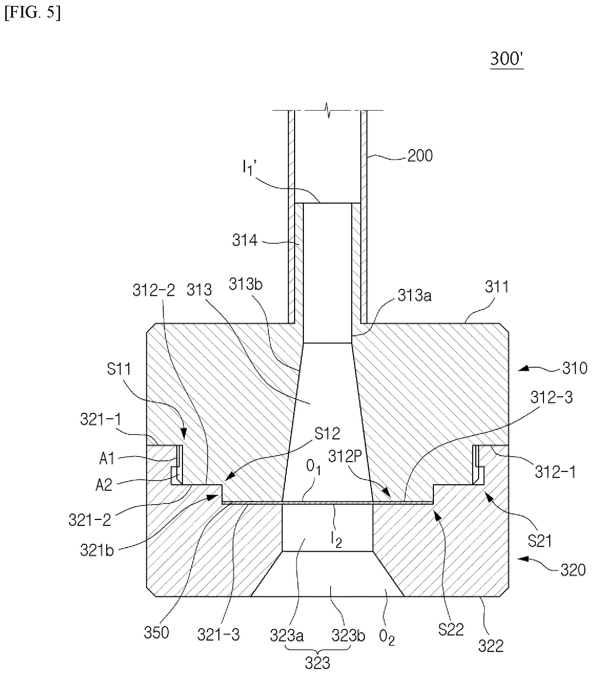

In an example embodiment, the upper kit part 310 and the lower kit part 320 may be coupled by fitting or screwing. For example, a fitting protrusion 312a may be provided on one of opposing surfaces of the upper kit part 310 and the lower kit part 320, an insertion groove 321a may be provided in the other surface, and the upper and lower kit parts 310 and 320 may be coupled to each other by inserting the fitting protrusion 312a into the insertion groove 321a (see FIGS. 2 and 3). Alternatively, screw threads A1 and A2 that are to be interlocked with each other are provided on the opposite surfaces of the upper kit part 310 and the lower kit part 320, and thus, the upper and lower kit parts 310 and 320 may be screw-coupled to each other (see FIGS. 4 and 5). Here, the opposite surfaces of the upper kit part 310 and the lower kit part 320 may be horizontal surfaces. Alternatively, as shown in FIGS. 4 and 5, when there is a step on the opposite surfaces of the upper kit part 310 and the lower kit part 320, the opposite surfaces may be vertical surfaces that are part of stepped surfaces. Accordingly, as shown in FIGS. 4 and 5, the upper and lower kit parts 310 and 320 may be screw-coupled to each other by providing the screw threads A1 and A2, which are to be interlocked with each other, on the opposite vertical surfaces of the upper and lower kit parts 310 and 320. Because the upper and lower kit parts 310 and 320 are coupled and the filter 350 is exposed only in a suspension permeation area (the first and second hollow channels 313 and 323), foreign substances are collected only in the suspension permeation area. In addition, the upper and lower kit parts 310 and 320 are firmly coupled by screws or the like and thus foreign substances do not diffuse outside a permeation area of the filter 350.

The filter 350 may be concentrically installed between the first outlet O1 and the second inlet I2 to entirely cover the first outlet O1 and the second inlet I2. In this case, an area of a foreign substance collection region 351 of the filter 350 is the same as an area of the first outlet O1 and the second inlet I2.

A receiving groove for accommodation of the filter 350 may be provided on at least one of the upper surface 321 of the lower kit part 320 and the lower surface 312 of the upper kit part 310. For example, a filter receiving groove 321b may be provided only in the upper surface 321 of the lower kit part 320, and the lower surface 312 of the upper kit part 310 opposite to the upper surface 321 may be a flat surface that merely covers the filter 350. Conversely, the filter receiving groove 312b may be provided only in the lower surface 312 of the upper kit part 310, and the upper surface 321 of the lower kit part 320 opposite to the lower surface 312 may be a flat surface that merely covers the filter 350. Alternatively, as shown in FIG. 3, the filter receiving grooves 312b and 321b for accommodation of the filter 350 may be provided in both the lower surface 312 of the upper kit part 310 and the upper surface 321 of the lower kit part 320. Due to such a receiving groove, the filter 350 may be stably placed between the upper and lower kit parts 310 and 320 and be fixed in the receiving groove without being moved when the upper and lower kit parts 310 and 320 are coupled to each other.

As another example, as shown in FIG. 5, the filter receiving groove 321b for accommodation of the filter 350 may be provided in one of the upper surface 321 of the lower kit part 320 and the lower surface 312 of the upper kit part 310, and a protrusion cover part 312P configured to cover the filter 350 when inserted into the filter receiving groove 321b may be provided on the other upper or lower surface 321 or 312. In this case, when the upper and lower kit parts 310 and 320 are coupled to each other, the filter 350 may be stably fixed and maintained in a gap between the filter receiving groove 321b and the protrusion cover part 312P.

A material of the foreign substance collection kit 300 may be a material with high chemical resistance, high wear resistance, and high strength. For example, the foreign substance collection kit 300 may formed of polyether ether keton (PEEK) with high chemical resistance and strength.

FIGS. 4 and 5 are a schematic view and a cross-sectional view of another example of a foreign substance collection kit 300' included in the foreign substance detecting device 10.

An injection tube 314 that communicates with a first hollow channel 313 and extends upward is provided on an upper surface 311 of an upper kit part 310 of the foreign substance collection kit 300'. The injection tube 314 protrudes upward and thus is easily coupled to an airtight conduit 200 as described above. For example, the injection tube 314 may be tightly fitted to a urethane hose to maintain a sealed state, and connect the airtight conduit 200 to the foreign substance collection kit 300'. In addition, the injection tube 314 of a predetermined length protrudes and is inserted into the airtight conduit 200 by a predetermined length, thereby preventing a risk of leakage of a suspension. In addition, the injection tube 314 extending upward functions as a funnel, thus facilitating injection of a suspension into the foreign substance collection kit 300' through an inlet I1' of the injection tube 314.

In the example of FIG. 3, first and second hollow channels 313 and 323 have the same diameter φ3. However, diameters of hollow channels may be changed according to heights thereof to help the flow of the suspension and the discharge of a liquid passing through a filter 350. For example, a bottom of at least one of the first hollow channel 313 and the second hollow channel 323 may be provided with tapered channel parts 313b and 323b whose channel cross-sectional areas increases toward an outlet of each of the first and second hollow channels 313 and 323.

As shown in FIG. 5, the first hollow channel 313 may include an upper channel 313a with a constant diameter and a lower channel 313b with a variable diameter. The upper channel 313a is connected to the injection tube 314 described above. A diameter of the upper channel 313a is set to be the same as an inner diameter of the injection tube 314 to ensure a stable downward flow of the suspension. Meanwhile, the lower channel 313b connected to the upper channel 313a has a tapered shape whose cross-sectional area gradually increases toward a first outlet O1. That is, the lower channel 313b is a tapered channel part 313b. Accordingly, the suspension flowing downward can be introduced into the filter 350 more smoothly and at a higher flow rate. In this case, a second inlet I2, a second hollow channel 323, and a second outlet O2 of the lower kit part 320 may have the same diameter as shown in FIG. 3. However, in this case, a diameter of the second inlet I2 and the like may be set to be the same as a diameter of the first outlet O1 of the tapered channel part 313b that expands.

Alternatively, as shown in FIG. 5, the second hollow channel 323 may include an upper channel 323a with a constant diameter and a lower channel 323b with a variable diameter. The upper channel 323a is connected to the first outlet O1 of the first hollow channel 313. The diameter of the upper channel 323a is set to a constant diameter to ensure a stable downward flow of the suspension. Meanwhile, the lower channel 323b connected to the upper channel 323a has a tapered shape whose cross-sectional area gradually increases toward the second outlet O2. That is, the lower channel 323b is a tapered channel part 323b. Accordingly, the filtered suspension containing the active material can be discharged more smoothly and at a higher flow rate.

FIG. 5 illustrates an example in which both the first hollow channel 313 and the second hollow channel 323 include tapered channel parts, but only one of the first hollow channel 313 and the second hollow channel 323 may include a tapered channel part.

The foreign substance collection kit 300' of FIGS. 4 and 5 includes a plurality of stepped parts S11, S12, S21, and S22 on opposite surfaces of the upper kit part 310 and the lower kit part 320. By combining the stepped parts S11, S12, S21, and S22, the upper and lower kit parts 310 and 320 may be coupled more firmly and the filter 350 may be more firmly fixed between the upper and lower kit parts 310 and 320. The upper kit part 310 includes two stepped parts on the lower surface 312 and thus a horizontal surface thereof facing the lower kit part 320 is divided into three horizontal surfaces 312-1, 312-2, and 312-3. In addition, the lower kit part 320 includes two stepped parts on the upper surface 321 and thus a horizontal face thereof facing the upper kit part 310 is divided into three horizontal surfaces 321-1, 321-2, and 321-3. A vertical surface is provided between adjacent horizontal surfaces among the three horizontal surfaces.

Referring to the drawings, a first lower surface stepped part S11 is provided on an outer side of the lower surface 312 of the upper kit part 310. In addition, a first upper surface stepped part S21 having a shape to be interlocked with the first lower surface stepped part S11 is provided on an outer side of the upper surface 321 of the lower kit part 320. The first lower surface stepped part S11 includes the two horizontal surfaces 312-1 and 312-2 and one vertical surface interposed therebetween, and the first upper surface stepped part S21 includes the two horizontal surfaces 321-1 and 321-2 and one vertical surface interposed therebetween. In this case, the opposite vertical surfaces of the first lower surface stepped part S11 and the first upper surface stepped part S21 may be provided with the screw threads A1 and A2 to be interlocked with each other and thus be screw-coupled to each other. Vertical lengths of screw portions on the vertical surfaces are determined by a distance by which the upper kit part 310 is moved downward according to screw coupling. As described below, the upper and lower kit parts 310 and 320 are coupled to each other such that the second lower surface stepped part S12 and the second upper surface stepped part S22 are interlocked with each other. In this case, the filter 350 between the second lower surface stepped part S12 and the second upper surface stepped part S22 may be damaged, when the second lower surface stepped part S12 is brought into extremely close contact with the second upper surface stepped part S22 and thus the second upper surface stepped part S22 is pressurized. Therefore, the vertical lengths of the screw portions are preferably set to be the same as a distance by which the upper kit part 310 is moved downward and a distance by which the second lower surface stepped part S12 is moved downward when screw coupling is performed, thereby preventing the filter 350 from being pressurized excessively.

The second lower surface stepped part S12 connected to the first lower surface stepped part S11 is provided on the inside of the lower surface 312 of the upper kit part 310. The second upper surface stepped part S22 connected to the first upper surface stepped part S21 and having a shape to be interlocked with the second lower surface stepped part S12 is provided on the inside of the upper surface 321 of the lower kit part 320. The filter 350 is installed between the opposite horizontal surfaces 312-3 and 321-3 of the second upper surface stepped part S22 and the second lower surface stepped part S12. Side surfaces of the filter 350 are regulated by the vertical surface of the second upper surface stepped part S22 to prevent movement of the filter 350. When the upper kit part 310 and the lower kit part 320 are screw-coupled to each other, the filter 350 is supported and fixed between the horizontal surfaces of the second lower surface stepped part S12 and the second upper surface stepped part S22 and by the vertical surface of the second upper surface stepped part S22. Accordingly, even when pressure is applied due to the downward flow of the suspension, the filter 350 may continuously filter foreign substances while being stably maintained.

For example, a filter seating sheet 360 coupled to the filter 350 while surrounding the filter 350 may be further provided and fixedly installed between the upper kit part 310 and the lower kit part 320.

The area of the foreign substance collection region 351 of the filter 350 may be set to an area to be scanned by an X-ray within a predetermined time according to a set resolution and scan speed of the analyzer 400 that analyzes foreign substances by scanning by X-rays.

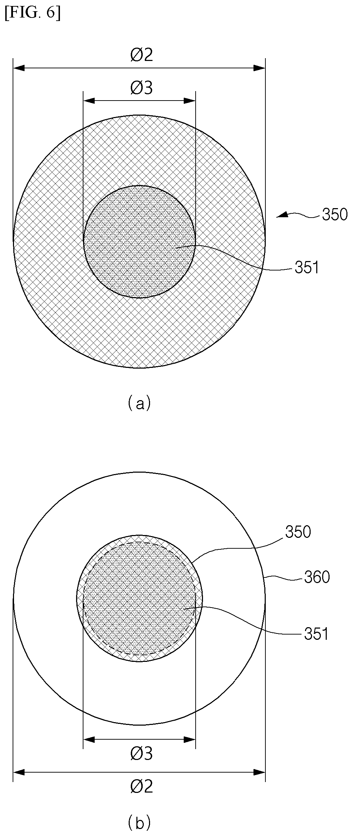

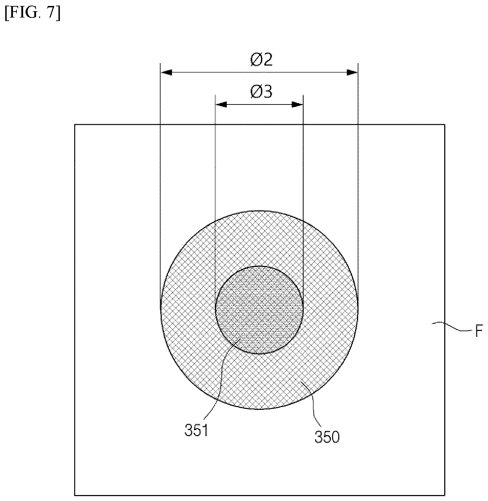

FIG. 6 is a schematic diagram illustrating an area of the foreign substance collection region 351 of the filter 350 applied to the present invention, and FIG. 7 is a schematic diagram illustrating a state in which the filter 350 is attached to a film to be inspected.

Conventionally, a dry method of filtering active material powder containing foreign substances using air pressure is employed, and therefore, the foreign substances are spread over a wide area due to the air pressure, thus making it impossible to increase a collection density of the foreign substances. Therefore, a cumbersome task of attaching a tape multiple times to collect foreign substances is needed. In this process, a loss rate of foreign substances is very high and thus the efficiency of collecting and detecting foreign substances is very low. In addition, foreign substances are detected together with an active material and thus are difficult to detect with a narrow X-ray scan area.

In contrast, according to the present invention, foreign substances are uniformly dispersed in the suspension tank 100 to prepare a suspension, and the suspension is continuously supplied to the filter 350, thus contributing to minimizing an area of the foreign substance collection region 351 of the filter 350. That is, foreign substances are uniformly dispersed not to be unequally distributed in the suspension, thereby preventing the filter 350 from being clogged during filtering. In addition, a suspension in which foreign substances are uniformly dispersed may be continuously supplied into the filter 350 having a pore size and thus capable of filtering foreign substances, so that only the foreign substances may be intensively collected in the filter 350. Foreign substances may be intensively collected in a small area by collecting the foreign substances by a wet method or wet pretreatment method as described above.

Referring to FIG. 6(a), a small central circle in a large outer circle represents a boundary of a region of the filter 350 in which foreign substances are collected. It can be seen that a diameter φ3 of the small central circle is much smaller than a diameter φ2 of the large outer circle. The diameter φ3 of the central circle is the same as a diameter φ3 of the first outlet O1 of the upper kit part 310 and the second inlet I2 of the lower kit part 320. In an example embodiment, the diameter φ3 of the central circle may be approximately 1 to 2 cm. However, the diameter φ3 and area of the foreign substance collection region 351 are not limited thereto and may be increased or reduced according to a resolution and scan speed of the analyzer 400. As described above, foreign substances may be inspected quickly by scanning a circle with a small area by X rays even at limited scanning speeds. In addition, a large amount of active material may be quickly inspected while satisfying a set resolution. As described above, according to the present invention, filtering may be performed by only a part of the filter 350 rather than the entire filter 350. A remaining part of the filter 350 that is not used for filtering is fixed between the upper and lower kit parts 310 and 320.

According to the present invention, because the area of the foreign substance collection region 351 may be minimized, an area of the filter 350 may be reduced to match the area of the foreign substance collection region 351. For example, as shown in FIG. 6(b), in an example embodiment, a filter seating sheet 360 coupled to the filter 350 with a small area while surrounding the filter 350 may be provided. The filter seating sheet 360 is a support sheet which the filter 350 is coupled to and seated on. When the filter seating sheet 360 is fixedly installed between the upper kit part 310 and the lower kit part 320, the filter 350 may be accurately positioned at a filtering location through which the suspension passes. In this case, a diameter of the filter 350 may be equal to or slightly greater than the diameter φ3 of the first outlet O1 of the upper kit part 310 and the second inlet I2 of the lower kit part 320.

FIG. 7 is a schematic view illustrating a state in which the filter 350 is attached to a film F to be inspected. For inspection using the analyzer 400, the filter 350 may be attached to the film F to be inspected, and the film F may be analyzed by being put into the analyzer 400. As shown in FIG. 7, a small central circular portion of the filter 350 may be scanned instead of scanning an entire area of the filter 350, thus greatly improving an inspection speed and efficiency.

According to the present invention, the analyzer 400 that analyzes the filter 350 in which foreign substances are collected to detect at least one of the types, a total number, shapes, and sizes of the foreign substances is provided. As described above, according to the present invention, it is possible to stack and collect a target foreign substance in a small area. Therefore, it can be expected that foreign substance detection efficiency will improve significantly regardless of the type of the analyzer 400.

An X-ray fluorescence (XRF) analyzer may be used as the analyzer 400. An XRF analysis is a method of analyzing a sample, which is to be inspected, by analyzing secondary fluorescence X-rays generated inside the sample when X-rays are emitted to the sample (or the sample is scanned by X-rays). Using the XRF analyzer 400, X-rays may be emitted to the filter 350 in which foreign substances are collected to qualitatively and quantitatively analyze the foreign substances from secondary fluorescence X-rays generated according to the type of the foreign substances. Fluorescent X-rays have different wavelengths and energies for each element. Therefore, the types of the foreign substances may be identified through qualitative analysis, based on wavelengths or energy of the secondary fluorescence X-rays.

In addition, the elements of the foreign substances or the number of the foreign substances may be identified according to a result of mapping analysis obtained based on a two-dimensional (2D) image obtained by X-ray scanning. For example, a mapping map in which pixels are shaded may be obtained by scanning the foreign substance collection region 351 by emitting X-rays thereto. The number of the foreign substances may also be identified by software with an algorithm that converts the shades in the mapping map into counts. Therefore, foreign substances in an electrode active material may be quantitatively analyzed.

Meanwhile, the shape and size of foreign substances may be identified using an optical microscope or electron microscope such as a scanning electron microscope (SEM) or a transmission electron microscope (TEM). Recently, an analyzer in which a microscope device such as an SEM is integrated into the XRF analyzer 400 has been released. Therefore, all the types, total number, shapes, and sizes of the foreign substances may be identified by such an analyzer. It is important to increase a degree of integration in collecting foreign substances and minimize the area of the foreign substance collection region 351 to improve foreign substance detection efficiency regardless of the type of an analyzer to be used, and the present invention is directed to achieving these tasks.

The foreign substance detecting device 10 of the present invention may set an area of the foreign substance collection region 351 of the filter 350 such that X-ray scanning performed by the XRF analyzer 400 may be completed within a predetermined time under set resolution and scan speed conditions of the XRF analyzer 400. That is, as shown in FIG. 6, the area of the foreign substance collection region 351 may be set to be small, and analysis may be completed within several to several tens of minutes (e.g., several minutes to thirty minutes) when a small area is scanned with the XRF analyzer 400 with a predetermined resolution and scan speed. Therefore, even when the above-described suspension and filtering times are included, only less than one hour is required to conduct analysis once, an electrode active material of a specific lot may be quickly inspected during a mass production process, thereby contributing to mass production.

Meanwhile, a shape of the foreign substance collection region 351 is not limited to a circular shape as shown in FIG. 6.

That is, when the shapes of the first inlet I1, the first hollow channel 313, and the first outlet O1 are changed to a rectangular shape, an elliptical shape, or other shapes, the shape of the foreign substance collection region 351 may be changed to a rectangular shape, an elliptical shape, or other shapes.

The shapes of the second inlet I2, the second hollow channel 323, and the second outlet O2 may also be changed to a rectangular shape, an elliptical shape, or other shapes.

### (Second Embodiment)

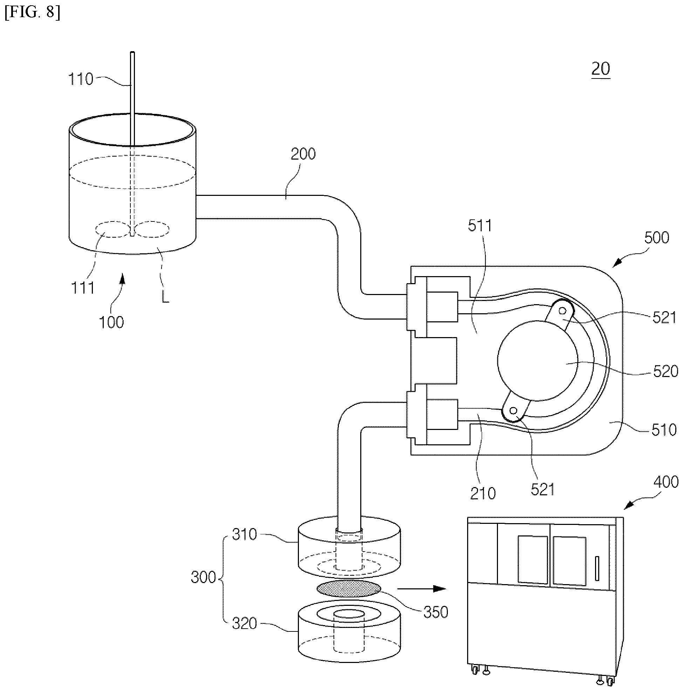

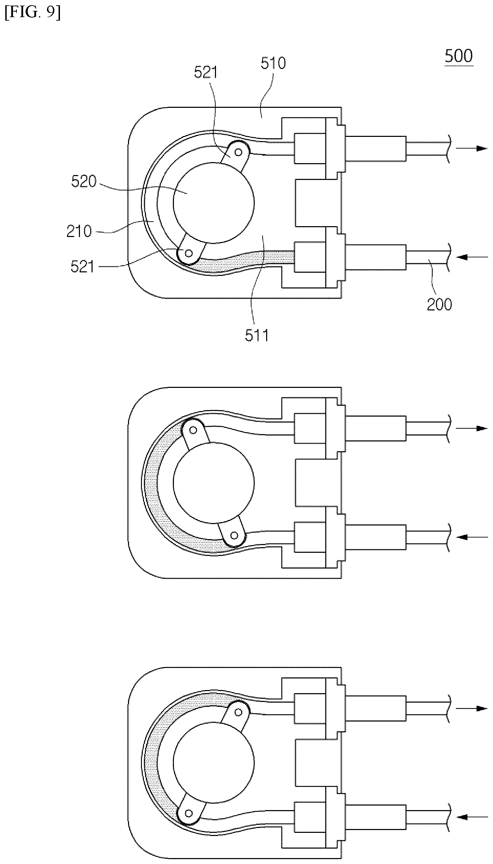

FIG. 8 is a schematic view of a device 20 capable of detecting foreign substances in an electrode active material according to a second embodiment of the present invention, and FIG. 9 is a schematic view illustrating an operation of a peristaltic pump. The second embodiment will be described focusing on differences from the first embodiment.

As in the first embodiment, the foreign substance detecting device 20 of the second embodiment includes a suspension tank 100, a foreign substance collection kit 300, and an analyzer 400. The second embodiment is different the first embodiment in that a peristaltic pump 500 capable of transferring a suspension to the foreign substance collection kit 300 at a constant flow rate is further provided. Referring to FIG. 8, the peristaltic pump 500 is installed in an airtight conduit 200 between the suspension tank 100 and the foreign substance collection kit 300 to transfer the suspension at a constant flow rate.

When a flow rate of the suspension transferred from the suspension tank 100 to a filter 350 changes greatly, pores of the filter 350 may be clogged due to locally concentrated foreign substances when filtering is performed by the filter 350. Therefore, it is necessary to transfer the suspension to the filter 350 at a constant flow rate if possible.

The peristaltic pump 500 is a positive displacement pump and is designed based on a peristaltic principle. The term "peristalsis" means a series of muscle contractions through which food is moved to different parts along the digestive organ. The peristaltic pump 500 moves a fluid in a manner similar to an action of a digestive organ.

Referring to FIG. 9, the peristaltic pump 500 includes a pump body 510 including therein a space 511 for accommodation of a fluid (suspension) conduit 200, and a rotation member 520 accommodated in the space 511. The rotation member 520 is rotated by a driving source such as a motor and includes rollers or shoes 521. A part 210 of the airtight conduit 200 is provided along an inner surface of the pump body 510 in the space 511. As shown in FIG. 9, the shoes 521 are installed at both ends of the rotation member 520, and the suspension may be transferred to the foreign substance collection kit 300 at a constant flow rate according to a direction of rotation of the shoes 521.

When the peristaltic pump 500 is used, only a tube (hose) comes into contact with a fluid, thus preventing contamination of the pump due to the suspension or contamination of the suspension due to the pump. In addition, when the pump is not in use, backflow is prevented and a check valve is not needed.

### <Foreign Substance Detecting Method>

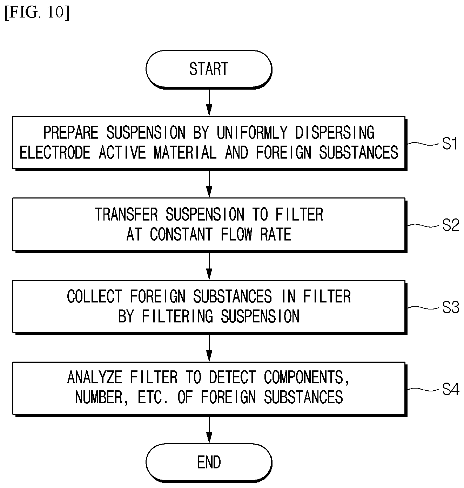

FIG. 10 is a flowchart of a method of detecting foreign substances in an electrode active material according to the present invention.

The method of detecting foreign substances according to the present invention may include: preparing a suspension in which an electrode active material is uniformly dispersed by suspending and agitating the electrode active material in a liquid (S1); collecting foreign substances contained in the electrode active material in a filter with a predetermined pore size by passing the suspension through the filter (S3); and analyzing the filter in which the foreign substances are collected to detect at least one of the types, total number, shapes, and sizes of the foreign substances (S4).

For example, foreign substances may be detected by the foreign substance detecting device 10 or 20 shown in FIG. 1 or 8.

First, the electrode active material is suspended and agitated in a liquid such as DI water or RO water. A positive electrode active material or a negative electrode active material may be used as the electrode active material. The method of the present embodiment will be described with respect to a case in which metal foreign substances larger than or equal to a specific size are filtered out of a positive electrode active material. For example, a large amount, e.g., 50 g, of a positive electrode active material is suspended in 1 L of DI water. A suspension may be prepared by agitating the active material in a suspension tank for five to ten minutes to be uniformly dispersed in a liquid.

The method of detecting foreign substances according to the present invention may further include transferring the suspension to the filter at a constant flow rate (S2). In this case, an airtight conduit may be used to transfer the suspension while being sealed from the outside. For example, a silicone tube (hose) or a urethane tube (hose) may be used as the airtight conduit. To transfer the suspension at a constant flow rate, the peristaltic pump 500 of FIG. 9 may be installed on the airtight conduit between the suspension tank and a foreign substance collection kit. For example, the suspension may be transferred from the suspension tank to the foreign substance collection kit 300 at a flow rate of 500 to 1000 ml/min through the peristaltic pump 500.

When the filter 350 is installed in the foreign substance collection kit 300 of FIGS. 2 and 4, filtering may be performed in an environment protected from the outside. For example, the filter 350 may be positioned between the upper kit part 310 and the lower kit part 320 and combined to fix the filter 350 between the upper and lower kit parts 310 and 320. When a remaining portion of the filter 350 excluding the foreign substance collection region 351 (or the filter seating sheet 360 of FIG. 6) is placed in a receiving groove and the upper and lower kit parts 310 and 320 are coupled to each other, foreign substances may be prevented from spreading outside the foreign substance collection region 351. That is, the foreign substance collection region 351 may be limited only to an area through which the suspension penetrates.

In addition, the foreign substance collection kit 300 is sealed except for an upper inlet (first inlet I1) of the upper kit part 310 and a lower outlet (second outlet O2) of the lower kit part 320, thus preventing contamination of the filter 350 and the like.

For example, in order to collect foreign substances with sizes of 40 to 65 µm, the filter 350 with the pore size corresponding to the sizes of the foreign substances may be used. For example, a polymer filter with a pore size of 40 µm may be used. Foreign substances in the electrode active material are collected in the filter 350 by passing the suspension through the filter 350 (S3). When the suction of the suspension by the peristaltic pump is completed, a filtered liquid is discharged sufficiently for a predetermined time period (e.g., one minute) through a connection tube connected to the first outlet O1. By discharging a filtrate for the time period, it is possible to secure a sufficient time for the filter 350 to sufficiently filter out foreign substances.

After filtering is completed, the filter 350 in which foreign substances are collected is analyzed to detect at least one of the types, a total number, shapes, and sizes of the foreign substances (S4).

After filtering is completed, the foreign substance collection kit 300 is disassembled to recover the filter 350. For inspection, the filter 350 may be attached to a film F to be inspected as in FIG. 7, and the film F may be put into the analyzer 400 to perform analysis.

The XRF analyzer 400 may be used to analyze foreign substances. The XRF analyzer 400 may emit X-rays to the filter 350 and qualitatively and quantitatively analyze foreign substances from secondary X-rays generated according to the types of the foreign substances. At least one of the types and number of foreign substances may be detected by the XRF analyzer 400. **In** addition, the shapes and sizes of the foreign substances may be detected by a microscope attached to the XRF analyzer 400 or an additional analyzer 400.

As described above, according to the present invention, an area of the foreign substance collection region 351 of the filter 350 may be minimized using wet pretreatment and filtering. To obtain a high-resolution image to be detected, a resolution of the XRF analyzer 400 is set to be within a predetermined range. **In** addition, a scan speed of the XRF analyzer 400 is determined. When the area of the foreign substance collection region 351 is small, X-ray scanning may be completed within a short period of time under the set resolution and scan speed conditions. Therefore, according to a foreign substance detection method of the present invention, an analysis speed of the XRF analyzer 400 may be increased and an analysis time may be reduced.

For example, it takes about 5 to 10 minutes to prepare the suspension and perform pretreatment for filtering, and XRF analysis may be performed within 30 minutes. For example, a large amount (e.g., about 100 g) of electrode active material may be analyzed. About 100 g of an active material may represent one intermediate product (lot). According to the present invention, a detection time can be reduced to inspect about 80 to 100 lots per day, thus meeting desired electrode and battery mass production rates.

In addition, according to the present disclosure, collection efficiency can be increased to achieve a very high detection rate of foreign substances. Therefore, the types, number, shapes, and sizes of foreign substances in the electrode active material can be detected with high probability. Accordingly, the quality of the electrode active material can be evaluated. For example, whether the electrode active material is appropriate or not may be determined according to the types and number of the foreign substances. When a battery is manufactured with a suitable electrode active material by evaluating the quality of the electrode active material as described above, a rate of defective battery cells may decrease greatly.

Accordingly, another aspect of the present invention may provide a secondary battery manufacturing method including evaluating the quality of an electrode active material according to a result of detecting foreign substances by the foreign substance detection method, and manufacturing a battery with the electrode active material selected according to a result of the evaluation.

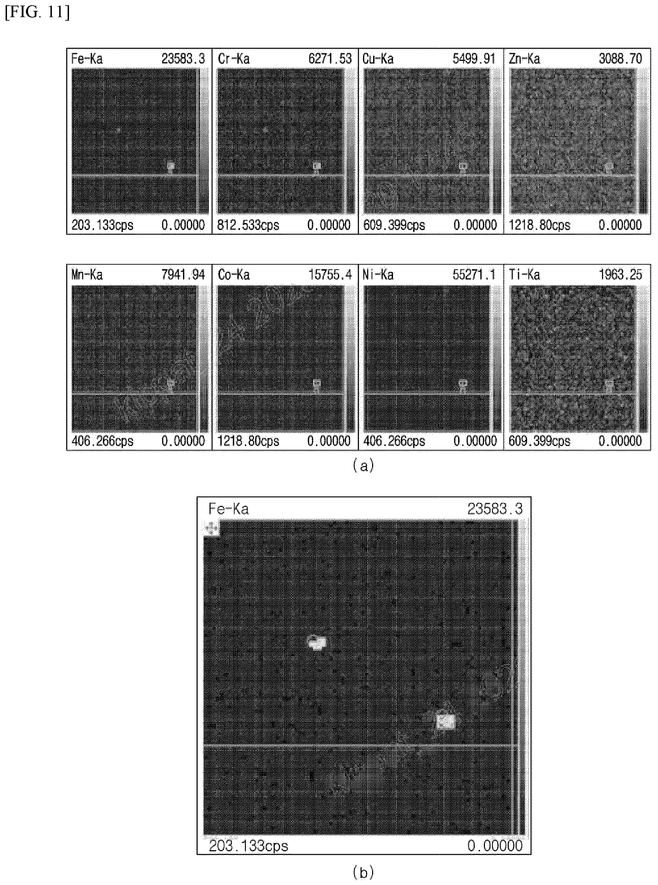

FIG. 11 is a mapping map for various foreign substances, obtained by an XRF analyzer.

A 2D image obtained from a wavelength of a secondary fluorescence X-ray was converted by software to obtain a mapping map of FIG. 11. It can be seen from FIG. 11 that there are various types of metal foreign substances, such as Fe, Cr, Cu, Zn, Mn, Co, Ni, and Ti, in a positive electrode active material according to the wavelength (see FIG. 11(a)). As described above, the types of foreign substances may be identified by the XRF analyzer 400. FIG. 11(b) is an enlarged view of a mapping map of Fe.

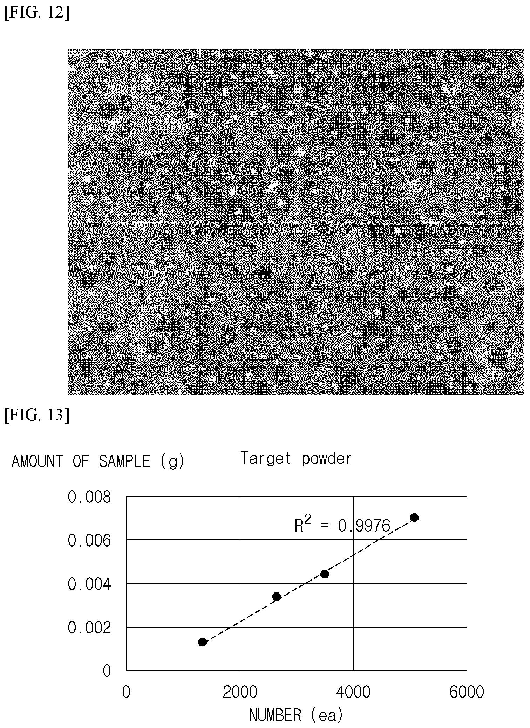

FIG. 12 is an image of foreign substances that is obtained by a microscope attached to the XRF analyzer 400. The shapes and sizes of the foreign substances may be calculated from the image of the foreign substances. The sizes of the foreign substances may be measured by comparing the size of the image with the sizes of the shapes of the foreign substances. A calculation program may be used in this case.

A process of detecting the number of foreign substances according to the present invention will be described with respect to examples below.

### [Embodiments of the Present Invention]

### <Examples>

100 g of NCM-based positive electrode active material powder was suspended in 1 L of DI water while changing an amount of foreign substance (Cu), and agitated in the suspension 100 of FIG. 1 for eight minutes to prepare a positive electrode active material suspension. Pressure was applied to the suspension by a peristaltic pump to transfer the suspension to a PEEK foreign substance collection kit at a flow rate of 500 ml/min. After the suction of the suspension is completed, a filtrate was drained for 60 seconds through a silicone tube connected to a lower portion of the foreign substance collection kit. A polyethylene filter with a pore size of 40 µm was used. A rectangular pipe of 15 mm×15 mm was used as the first hollow channel 313, thereby minimizing an area of the foreign substance collection region 351 to 15 mm×15 mm.

The foreign substance collection kit was disassembled, and the filter was analyzed by being put into an XRF FID analyzer (EA8000, manufactured by Hitachi Co., Ltd.) while being attached to a film to be inspected.

X-rays were emitted from the XRF analyzer to a scan size of 15 mm×15 mm by a mapping scanning method. A resolution was set to 30 µm/pixel, and a scanning speed was set to 5 ms.

FIG. 13 is a graph showing a correlation between the amount of foreign substance powder and the number of foreign substances.

FIG. 13 illustrates a correlation between the weight and number of coppers. The correlation represents that there is a certain relationship between two variables, and a correlation coefficient indicating a measure of the correlation may be calculated. A statistical method of calculating a correlation coefficient from two pieces of variable data is well known and thus a detailed description thereof is omitted here.

FIG. 13 shows a correlation coefficient between the number and weight of coppers contained in the same type of positive electrode active material as an NCM-based positive electrode active material used for XRF analysis. As shown in FIG. 13, the correlation coefficient was 0.9976 that is almost close to 1. Thus, it can be estimated that 2338 Cu powders are contained in 0.003 g of Cu.

**[Table 1]**

| Experimental Example | Positive electrode active material D50 (µm) | Positive electrode active material Dmax (µm) | Foreign substance size (µm) | Input amount (g) of foreign substances | Number of foreign substances measured by XRF | Number of foreign substances, converted based on 0.003 g | Detection rate (recovery rate) of foreign substances |
|---|---|---|---|---|---|---|---|
| 1 | 10.2 to 11.6 | 27.5 | 53 to 75 | 0.0034 | 2050 | 1809 | 77% |

| | | | | | | | |
|---|---|---|---|---|---|---|---|
| 2 | 10.2 to 11.6 | 27.5 | 53 to 75 | 0.0033 | 2164 | 1957 | 84% |
| 3 | 10.2 to 11.6 | 27.5 | 53 to 75 | 0.0034 | 2341 | 2066 | 88% |

It can be seen from Table 1 that median particle size D50 of the positive electrode active material is 10.2 to 11.6 µm, and maximum particle size thereof is 27.5 µm, and thus, the positive electrode active material is drained through the filter. The numbers of coppers in Experimental Examples 1 to 3 are 2050, 2164, and 2341. To compare the numbers of coppers under the same criterion, they are converted into 1809, 1967, and 2066, based on 0.003 g of Cu.

Referring to FIG. 13, 2338 powders are contained in 0.003 g of Cu and thus foreign substance detection rates, i.e., recovery rates of foreign substances recovered from injected foreign substances, in Examples 1 to are 77%, 84%, and 88%, respectively.

Therefore, according to the foreign substance detection method of the present invention, foreign substances can be detected with a high probability of 83% on average.

As described above, according to a foreign substance detecting device and method of the present invention, foreign substances in an electrode active material can be detected quickly with a high probability, thus greatly increasing detection reliability.

The above description is only an example of the technical idea of the present invention and various modification and changes may be made by those of ordinary skill in the technical field to which the present invention pertains without departing from the essential features of the present invention.

Therefore, the drawings of the present invention set forth herein are intended not to limit the technical idea of the present invention but to describe the technical idea, and the scope of the technical idea of the present invention is not limited by the drawings. The scope of protection for the present invention should be interpreted based on the following claims and all technical ideas within the same scope as the present invention should be interpreted as being included in the scope of the present invention.

### (Reference Numerals)

10, 20: foreign substance detecting device
100: suspension tank
L: suspension
110: agitating member
200: airtight conduit
300, 300': foreign substance collection kit
310: upper kit part
320: lower kit part
350: filter
351: foreign substance collection region
360: filter seating sheet
400: (XRF) analyzer
F: film to be inspected
500: peristaltic pump

## Claims

1. A device for detecting foreign substances in an electrode active material, the device comprising:
a suspension tank containing a suspension in which an electrode active material is uniformly dispersed;
a filter having a predetermined pore size, and configured to filter the suspension transferred from the suspension tank to collect foreign substances in the electrode active material; and
an analyzer configured to analyze the filter in which the foreign substances are collected to detect at least one of types, a total number, shapes, and sizes of the foreign substances.

2. The device of claim 1, wherein the suspension tank comprises an agitating member configured to agitate and disperse the electrode active material and the foreign substances.

3. The device of claim 1, wherein the suspension is transferred to the filter at a constant flow rate.

4. The device of claim 1, wherein the suspension is transferred to the filter and filtered by the filter while being sealed from the outside.

5. The device of claim 1, further comprising a foreign substance collection kit including a liquid inlet, a liquid outlet, and a communication channel connecting the liquid inlet and the liquid outlet, the foreign substance collection kit being sealed from the outside except for the liquid inlet and the liquid outlet,
wherein the filter is installed in the communication channel.

6. The device of claim 5, wherein the liquid inlet of the foreign substance collection kit and the suspension tank are connected through an airtight conduit.

7. The device of claim 6, further comprising a peristaltic pump installed in the sealed conduit between the suspension tank and the foreign substance collection kit to transfer the suspension at a constant flow rate.

8. The device of claim 1, wherein the filter comprises a polymer filter.

9. The device of claim 1, wherein the analyzer comprises an X-ray fluorescence (XRF) analyzer configured to analyze the foreign substances qualitatively and quantitatively from secondary X-rays generated for types of the foreign substances by emitting X-rays to the filter in which the foreign substances are collected.

10. The device of claim 9, wherein an area of a foreign substance collection region of the filter is set to complete X-ray scanning by the X-ray fluorescence analyzer within a predetermined time under set resolution and scan speed conditions of the X-ray fluorescence analyzer.

11. A method of detecting foreign substances in an electrode active material, the method comprising:
preparing a suspension by suspending and agitating an electrode active material in a liquid, wherein the electrode active material is uniformly dispersed in the suspension;
collecting foreign substances, which are contained in the electrode active material, in the filter by passing the suspension through a filter with a predetermined pore size; and
analyzing the filter in which the foreign substances are collected to detect at least one of types, a total number, shapes, and sizes of the foreign substances.

12. The method of claim 11, wherein the suspension is transferred to the filter at a constant flow rate.

13. The method of claim 11, wherein the suspension is transferred to the filter and filtered by the filter while being sealed from the outside.

14. The method of claim 11, wherein at least one of types and total number of the foreign substances is detected by an X-ray fluorescence analyzer that analyzes the foreign substances qualitatively and quantitatively from secondary X-rays generated for the types of the foreign substances by emitting X-rays to the filter in which the foreign substances are collected.

15. The method of claim 14, wherein an area of a foreign substance collection region of the filter is set to complete X-ray scanning by the X-ray fluorescence analyzer within a predetermined time under set resolution and scan speed conditions of the X-ray fluorescence analyzer.
